# EUROPEAN PATENT APPLICATION

(11) **EP 1 930 431 A1**
(43) Date of publication of application: **11.06.2008**
(21) Application number: 06797575.5
(22) Date of filing: 31.08.2006
(51) Int. Cl.: C12N 15/09, C07K 14/395, C12C 11/02, C12G 1/00, C12G 3/02, C12N 1/19, C12Q 1/04, C12Q 1/68

(54) **GENE CAPABLE OF ENHANCING LOW TEMPERATURE FERMENTATION ABILITY AND/OR FREEZING STRESS RESISTANCE AND USE THEREOF**

(30) Priority: 01.09.2005 JP 2005253250
(71) Applicant: SUNTORY LIMITED, Osaka-shi, Osaka 530-8203 (JP)
(72) Inventor: NAKAO, Yoshihiro, Mishima-gun Osaka 618-8503 (JP); KODAMA, Yukiko, Mishima-gun Osaka 618-8503 (JP); SHIMONAGA, Tomoko, Mishima-gun Osaka 618-8503 (JP)
(74) Representative: Vossius & Partner
(86) International application number: PCT/JP2006/317699
(87) International publication number: WO 2007/026956

(57) **Abstract**

The present invention relates to a gene capable of improving low temperature performance (low temperature fermentability and/or low temperature resistance) and use thereof, in particular, to a brewery yeast with superior low temperature performance, alcoholic beverages produced using said yeast, and a method for producing said beverages. More particularly, the present invention relates to a yeast, whose low temperature performance is improved by amplifying expression level of DLT1 gene encoding Dltlp capable of improving low temperature performance of a brewery yeast, especially non-ScDLT1 gene specific to a lager brewing yeast, and to a method for producing alcoholic beverages using said yeast.

## Description

### TECHNICAL FIELD

The present invention relates to a gene capable of improving low temperature fermentability and/or low temperature resistance (hereinafter also referred to as "low temperature performance") and use thereof, in particular, to a brewery yeast with superior low temperature performance, alcoholic beverages produced using said yeast, and a method for producing said beverages. More particularly, the present invention relates to a yeast, whose low temperature performance is improved by amplifying expression level of DLT1 gene encoding Dltlp that is a protein capable of improving low temperature performance of a brewery yeast, especially non-ScDLT1 gene specific to a lager brewing yeast, and to a method for producing alcoholic beverages using said yeast.

### BACKGROUND ART

Lager beer is produced by fermentation at a low temperature (10 to 15°C) and has refreshing taste without harsh flavor as its characteristic feature. Bottom fermentation yeasts, which are used for producing lager beer, have superior low temperature fermentability. However, a gene involved in low temperature fermentability has not been revealed.

Further, with respect to sake, which is also produced by fermentation at a low temperature, it is known that a low temperature plays an important role in maintaining the activity of flavor component-producing enzymes such as esters, reducing the activity of flavor component-degrading enzymes, increasing flavor component substrates, and the like.

Domestic Publication of PCT International Publication No. 97/02444 reports an example in which low temperature fermentability was improved by expression of a gene complementary to mutation showing cold sensitivity of fermentability. Further, LTG3 (DLT1) has been reported as a gene involved in low temperature growth (Studies of sake yeasts, Studies in 1990s, Society for the Study of Sake Yeast and Gluten Ed., pp. 103-107,2003).

As genes capable of improving low temperature resistance of baker's yeasts, YLR023c and YMR126c (DLT1) have been reported (Japanese Patent Application Laid-Open No. 2003-144137).

### DISCLOSURE OF INVENTION

Under the above-described circumstances, a yeast having superior low temperature fermentability has been desired to produce alcoholic beverages having excellent flavor at a low temperature. Further, productivity would be improved if yeasts can be stably preserved for a long period of time by freezing. Therefore, a yeast having superior low temperature resistance is also desired.

To solve the problems described above, the present inventors made extensive studies, and as a result succeeded in identifying and isolating a gene encoding a protein capable of improving low temperature performance and attaining more advantageous effects compared to known proteins from a lager brewing yeast. Moreover, a yeast in which the obtained gene was transformed and expressed was produced to confirm improvement in low temperature performance, thereby completing the present invention.

Thus, the present invention relates to a novel gene capable of improving low temperature performance characteristically existing in a lager brewing yeast, to a protein encoded by said gene, to a transformed yeast in which the expression of said gene is controlled, and to a method for producing alcoholic beverages using a yeast in which the expression of said gene is controlled. More specifically, the present invention provides the following polynucleotides, a vector comprising said polynucleotide, a transformed yeast introduced with said vector, a method for producing alcoholic beverages by using said transformed yeast, and the like.
(1) A polynucleotide selected from the group consisting of:
   (a) a polynucleotide comprising a polynucleotide consisting of the nucleotide sequence of SEQ ID NO:1;
   (b) a polynucleotide comprising a polynucleotide encoding a protein consisting of the amino acid sequence of SEQ ID NO:2;
   (c) a polynucleotide comprising a polynucleotide encoding a protein consisting of the amino acid sequence of SEQ ID NO:2 with one or more amino acids thereof being deleted, substituted, inserted and/or added, and having an activity to improve low temperature performance;
   (d) a polynucleotide comprising a polynucleotide encoding a protein having an amino acid sequence having 60% or higher identity with the amino acid sequence of SEQ ID NO:2, and having an activity to improve low temperature performance;
   (e) a polynucleotide comprising a polynucleotide which hybridizes to a polynucleotide consisting of a nucleotide sequence complementary to the nucleotide sequence of SEQ ID NO:1 under stringent conditions, and which encodes a protein having an activity to improve low temperature performance; and
   (f) a polynucleotide comprising a polynucleotide which hybridizes to a polynucleotide consisting of a nucleotide sequence complementary to the nucleotide sequence of the polynucleotide encoding the protein of the amino acid sequence of SEQ ID NO:2 under stringent conditions, and which encodes a protein having an activity to improve low temperature performance.
(2) The polynucleotide of (1) above selected from the group consisting of:
   (g) a polynucleotide comprising a polynucleotide encoding a protein consisting of the amino acid sequence of SEQ ID NO: 2, or an amino acid sequence of SEQ ID NO: 2 wherein 1 to 10 amino acids thereof is deleted, substituted, inserted, and/or added, and wherein said protein has an activity to improve low temperature performance;
   (h) a polynucleotide comprising a polynucleotide encoding a protein having an amino acid sequence which has 90% or higher identity with the amino acid sequence of SEQ ID NO: 2, and having an activity to improve low temperature performance; and
   (i) a polynucleotide comprising a polynucleotide consisting of the nucleotide sequence of SEQ ID NO: 1 or a polynucleotide which hybridizes to a polynucleotide consisting of a nucleotide sequence complementary to the nucleotide sequence of SEQ ID NO: 1 under high stringent conditions, and which encodes a protein having an activity to improve low temperature performance.
(3) The polynucleotide of (1) above comprising a polynucleotide consisting of SEQ ID NO: 1.
(4) The polynucleotide of (1) above comprising a polynucleotide encoding a protein consisting of SEQ ID NO: 2.
(5) The polynucleotide of any one of (1) to (4) above, wherein the polynucleotide is DNA.
(6) A protein encoded by the polynucleotide of any one of (1) to (5) above.
(7) A vector comprising the polynucleotide of any one of (1) to (5) above.
   (7a) The vector of (7) above, which comprises the expression cassette comprising the following components:
   (x) a promoter that can be transcribed in a yeast cell;
   (y) any of the polynucleotides described in (1) to (5) above linked to the promoter in a sense or antisense direction; and
   (z) a signal that can function in a yeast with respect to transcription termination and polyadenylation of a RNA molecule.
(8) A yeast, wherein the vector of (7) above is introduced.
(9) The yeast of (8) above, whose low temperature performance is improved by introducing the vector of (7) above.
(10) The yeast of (9) above, whose low temperature performance is improved by increasing the expression level of the protein of (6) above.
(11) A method for producing an alcoholic beverage comprising culturing the yeast of any one of (8) to (10) above.
(12) The method for producing an alcoholic beverage of (11) above, wherein the brewed alcoholic beverage is a malt beverage.
(13) The method for producing an alcoholic beverage of (11) above, wherein the brewed alcoholic beverage is wine.
(14) An alcoholic beverage produced by the method of any one of (11) to (13) above.
(15) A method for assessing a test yeast for its low temperature performance, comprising using a primer or a probe designed based on a nucleotide sequence of a gene having the nucleotide sequence of SEQ ID NO: 1 and capable of improving low temperature performance.
   (15a) A method for selecting a yeast having superior low temperature performance by using the method described in (15) above.
   (15b) A method for producing an alcoholic beverage (for example, beer) by using the yeast selected with the method in (15a) above.
(16) A method for assessing a test yeast for its low temperature performance, comprising: culturing a test yeast; and measuring an expression level of a gene having the nucleotide sequence of SEQ ID NO: 1 and capable of improving low temperature performance.
(17) A method for selecting a yeast, comprising: culturing test yeasts; quantifying the protein according to (6) above or measuring an expression level of a gene having the nucleotide sequence of SEQ ID NO: 1 and capable of improving low temperature performance; and selecting a test yeast having said protein amount or said gene expression level according to a target low temperature performance.
   (17a) A method for selecting a yeast, comprising: culturing test yeasts; measuring low temperature fermentability or low temperature fermentation activity, or low temperature resistance in each yeast; and selecting a test yeast having a target low temperature fermentability or low temperature fermentation activity, or low temperature resistance.
(18) The method for selecting a yeast according to (17) above, comprising: culturing a reference yeast and test yeasts; measuring an expression level of a gene having the nucleotide sequence of SEQ ID NO: 1 and capable of improving low temperature performance in each yeast; and selecting a test yeast having the gene expressed higher than that in the reference yeast.
(19) The method for selecting a yeast according to (17) above, comprising: culturing a reference yeast and test yeasts; quantifying the protein according to (6) above in each yeast; and selecting a test yeast having said protein in a larger amount than that in the reference yeast. That is, the method for selecting a yeast described in (17) above comprising: culturing plural yeasts; quantifying the protein of (6) above in each yeast; and selecting a test yeast having a large amount of the protein from them.
(20) A method for producing an alcoholic beverage comprising: conducting fermentation for producing an alcoholic beverage using the yeast according to any one of (8) to (10) above or a yeast selected by the method according to any one of (17) to (19) above; and improving low temperature performance.

According to the method for producing alcoholic beverages using the transformed yeast of the present invention, low temperature fermentability is improved and fermentation period of low temperature fermentation can be shortened. Further, according to the present invention, a yeast having superior low temperature resistance can be provided.

As used herein, "low temperature performance" refers to low temperature fermentability and/or low temperature resistance.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract consumption with time upon beer fermentation test. The horizontal axis represents fermentation time while the vertical axis represents apparent extract concentration (w/w%).
Figure 3 shows the expression behavior of non-ScDLT1 gene in yeasts upon beer fermentation test. The horizontal axis represents fermentation time while the vertical axis represents the brightness of detected signal.
Figure 4 shows the degrees of low temperature resistance of the parent strain and the non-ScDLT1 highly expressed strain.

### BEST MODES FOR CARRYING OUT THE INVENTION

The present inventors conceived that it is possible to conduct low temperature fermentation more efficiently by improving low temperature performance of yeasts. The present inventors have studied based on this conception and as a result, isolated and identified a non-ScDLT1 gene encoding a protein capable of improving low temperature performance unique to lager brewing yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide consisting of the nucleotide sequence of SEQ ID NO:1; and (b) a polynucleotide comprising a polynucleotide encoding a protein consisting of the amino acid sequence of SEQ ID N0:2. The polynucleotide can be DNA or RNA.

The target polynucleotide of the present invention is not limited to the polynucleotide encoding a protein capable of improving low temperature performance derived from lager brewing yeast described above and may include other polynucleotides encoding proteins having equivalent functions to said protein. Proteins with equivalent functions include, for example, (c) a protein consisting of an amino acid sequence of SEQ ID NO: 2 with one or more amino acids thereof being deleted, substituted, inserted and/or added and having an activity to improve low temperature performance.

Such proteins include a protein consisting of an amino acid sequence of SEQ ID NO: 2 with, for example, 1 to 100, 1 to 90, 1 to 80, 1 to 70, 1 to 60, 1 to 50, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and having an activity to improve low temperature performance. In general, the number of deletions, substitutions, insertions, and/or additions is preferably smaller. In addition, such proteins include (d) a protein having an amino acid sequence with about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having an activity to improve low temperature performance. In general, the percentage identity is preferably higher.

Low temperature fermentability can be assessed, for example, by measuring the ethanol production amount and the rate of ethanol production at 10 to 15 °C. When brewing at the same temperature, if the ethanol production amount or the rate of ethanol production in the case of a test yeast is increased compared to the case of a reference yeast (e.g., *Saccharomyces cerevisiae* NBRC2002, AJL4002 or the like), it is judged that the test yeast has an "activity to improve low temperature fermentability". Such increasing rate is preferably 5% or higher, more preferably 10% or higher, even more preferably 15% or higher, and still more preferably 20% or higher. Low temperature resistance can be assessed, for example, by comparing the rate of glucose consumption in the case, where a yeast suspension containing a test yeast is not frozen, with that in the case, where the yeast suspension containing the test yeast is once frozen and then thawed, like the method described in Example 5 of the present specification. That is, the smaller the decrease in the rate of glucose consumption after freezing, the higher the low temperature resistance.

Furthermore, the present invention also encompasses (e) a polynucleotide comprising a polynucleotide which hybridizes to a polynucleotide consisting of a nucleotide sequence complementary to the nucleotide sequence of SEQ ID NO: 1 under stringent conditions and which encodes a protein having an activity to improve low temperature performance; and (f) a polynucleotide comprising a polynucleotide which hybridizes to a polynucleotide consisting of a nucleotide sequence complementary to a nucleotide sequence of a polynucleotide encoding a protein consisting of the amino acid sequence of SEQ ID NO: 2 under stringent conditions, and which encodes a protein having an activity to improve low temperature performance.

Herein, "a polynucleotide that hybridizes under stringent conditions" refers to a polynucleotide, such as a DNA, obtained by a colony hybridization technique, a plaque hybridization technique, a southern hybridization technique or the like using all or a part of a polynucleotide consisting of a nucleotide sequence complementary to the nucleotide sequence of SEQ ID NO: 1 or a polynucleotide encoding the amino acid sequence of SEQ ID NO: 2 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions and high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1 % or higher, 99.2% or higher, 99.3% or higher, 99.4% or higher, 99.5% or higher, 99.6% or higher, 99.7% or higher, 99.8% or higher or 99.9% or higher identity to a polynucleotide encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad. Sci. USA, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

### 2. Protein of the present invention

The present invention also provides proteins encoded by any of the polynucleotides (a) to (i) above. A preferred protein of the present invention comprises an amino acid sequence of SEQ ID NO:2 with one or several amino acids thereof being deleted, substituted, inserted and/or added, and has an activity to improve low temperature performance. Such protein includes those having an amino acid sequence of SEQ ID NO: 2 with amino acid residues thereof of the number mentioned above being deleted, substituted, inserted and/or added and having an activity to improve low temperature performance. In addition, such protein includes those having homology as described above with the amino acid sequence of SEQ ID NO: 2 and having an activity to improve low temperature performance. Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad. Sci. USA 79: 6409 (1982), Gene 34: 315 (1985), Nuc. Acids. Res., 13: 4431 (1985), Proc. Natl. Acad. Sci. USA 82: 488 (1985).

Deletion, substitution, insertion and/or addition of one or more amino acid residues in an amino acid sequence of the protein of the invention means that one or more amino acid residues are deleted, substituted, inserted and/or added at any one or more positions in the same amino acid sequence. Two or more types of deletion, substitution, insertion and addition may occur concurrently.

Hereinafter, examples of mutually substitutable amino acid residues are enumerated. Amino acid residues in the same group are mutually substitutable. The groups are provided below.
Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides (DNA) described in (a) to (i) above. Generally, the vector of the present invention comprises an expression cassette including as components (x) a promoter that can transcribe in a yeast cell; (y) a polynucleotide (DNA) described in any of (a) to (i) above that is linked to the promoter in a sense or antisense direction; and (z) a signal that functions in the yeast with respect to transcription termination and polyadenylation of RNA molecule.

A vector introduced in the yeast may be any of a multicopy type (YEp type), a single copy type (YCp type), or a chromosome integration type (YIp type). For example, YEp24 (J. R. Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., *EMBO* J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method *(*Meth. Enzym., 194: 182 (1990)), a spheroplast method *(*Proc. Natl. Acad. Sci. USA, 75: 1929(1978)), a lithium acetate method *(*J. Bacteriology, 153: 163 (1983)), and methods described in Proc. Natl. Acad. Sci. USA, 75: 1929 (1978), METHODS IN YEAST GENETICS, 2000 Edition: A Cold Spring Harbor Laboratory Course Manual, and the like.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to produce an alcoholic product at a low temperature over a short period of time. In addition, yeasts to be selected by the yeast assessment method of the present invention described below can also be used. The target alcoholic beverages include, for example, but not limited to beer, beer-taste beverages such as sparkling liquor (*happoushu*), wine, whisky, sake and the like.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages whose fermentation period is shortened. Thus, according to the present invention, alcoholic beverages can be produced using the existing facility without increasing the cost.

### 5. Yeast assessment method of the invention

The present invention relates to a method for assessing a test yeast for its low temperature performance by using a primer or a probe designed based on a nucleotide sequence of a gene having the nucleotide sequence of SEQ ID NO:1 and capable of improving low temperature performance. General techniques for such assessment method are known and are described in, for example, WO01/040514, Japanese Laid-Open Patent Application No. 8-205900 or the like. This assessment method is described below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the gene capable of improving low temperature performance, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of a polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the low temperature performance of the yeast as determined by whether the molecular weight of the amplified product is a size that contains the DNA molecule of the specific part. In addition, by analyzing the nucleotide sequence of the amplified product, the above-described ability may be predicted and/or assessed more precisely.

Moreover, in the present invention, a test yeast is cultured to measure an expression level of the gene having the nucleotide sequence of SEQ ID NO: 1 and capable of improving low temperature performance to assess the test yeast for its low temperature performance. In measuring an expression level of the gene, the test yeast is cultured and then mRNA or a protein resulting from the transcription of the gene capable of improving low temperature performance is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the gene having the nucleotide sequence of SEQ ID NO: 1 and capable of improving low temperature performance are measured to select a test yeast with the gene expression level corresponding to the target low temperature performance, thereby selecting a yeast favorable for brewing desired alcoholic beverages. In addition, a reference yeast and test yeasts may be cultured so as to measure and compare the expression level of the gene in each of the yeasts, thereby selecting a favorable test yeast. More specifically, for example, a reference yeast and test yeasts are cultured and an expression level of the gene having the nucleotide sequence of SEQ ID NO: 1 and capable of improving low temperature performance is measured in each yeast. By selecting a test yeast with the gene expressed higher than that in the reference yeast, a yeast suitable for brewing desired alcoholic beverages can be selected.

Alternatively, test yeasts are cultured and a yeast with a superior low temperature performance is selected, thereby selecting a yeast suitable for brewing desired alcoholic beverages.

In these cases, the test yeasts or the reference yeast may be, for example, a yeast introduced with the vector of the invention, a yeast in which an expression of the polynucleotide (DNA) of the invention described above has been suppressed, an artificially mutated yeast or a naturally mutated yeast. The low temperature fermentability can be assessed, for example, by measuring the ethanol production amount and the rate of ethanol production at 10 to 15 °C. The low temperature resistance can be assessed, for example, by the method described in Example 5. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (see, e.g., Yasuji Oshima Ed., Biochemistry Experiments vol. 39, Yeast Molecular Genetic Experiments, pp. 67-75, JSSP).

In addition, examples of yeasts used as the reference yeast or the test yeasts include any yeast that can be used for brewing, for example, brewery yeasts for beer, wine, sake and the like. More specifically, yeasts such as genus *Saccharomyces* may be used. According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Novel Gene Capable of Improving Low Temperature Fermentability (non-ScDLT1)

A specific novel gene capable of improving low temperature fermentability (non-ScDLT1) (SEQ ID NO: 1) from a lager brewing yeast was found, as a result of a search utilizing the comparison database described in Japanese Patent Application Laid-Open No. 2004-283169. Based on the acquired nucleotide sequence information, primers non-ScDLT1_F (SEQ ID NO: 3) and non-ScDLT1_R (SEQ ID NO: 4) were designed to amplify the full-length genes, respectively. PCR was carried out using chromosomal DNA of a genome sequencing strain, *Saccharomyces pastorianus* Weihenstephan 34/70 strain, as a template to obtain DNA fragments including the full-length gene of non-ScDLT1.

The thus-obtained non-ScDLT1 gene fragment was inserted into pCR2.1-TOPO vector (Invitrogen) by TA cloning. The nucleotide sequences of non-ScDLT1 gene were analyzed according to Sanger's method (F. Sanger, Science, 214: 1215, 1981) to confirm the nucleotide sequence.

### Example 2: Analysis of Expression of non-ScDLT1 Gene during Beer Fermentation

A beer fermentation test was conducted using a lager brewing yeast, *Saccharomyces pastorianus* W34/70 strain and then mRNA extracted from yeast cells during fermentation was analyzed by a DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

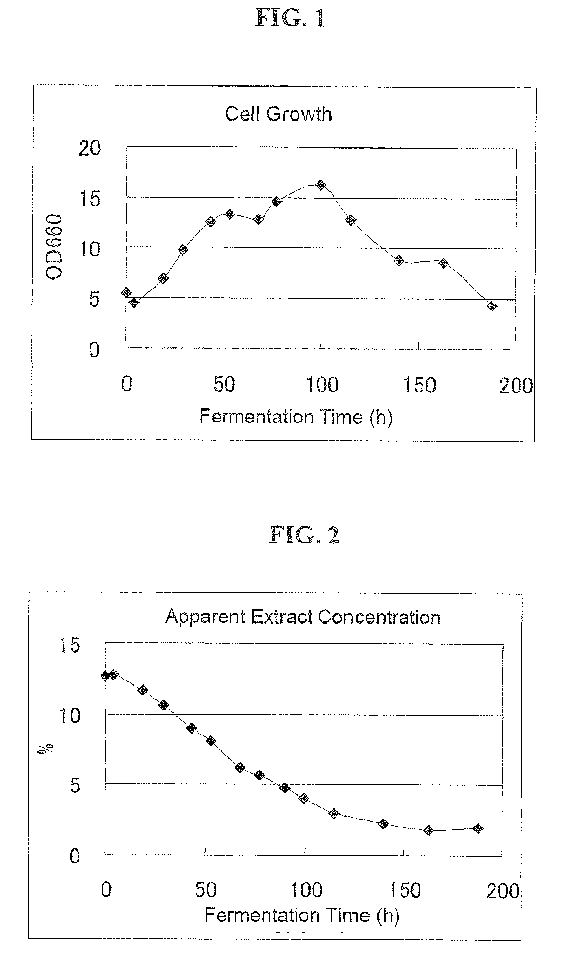

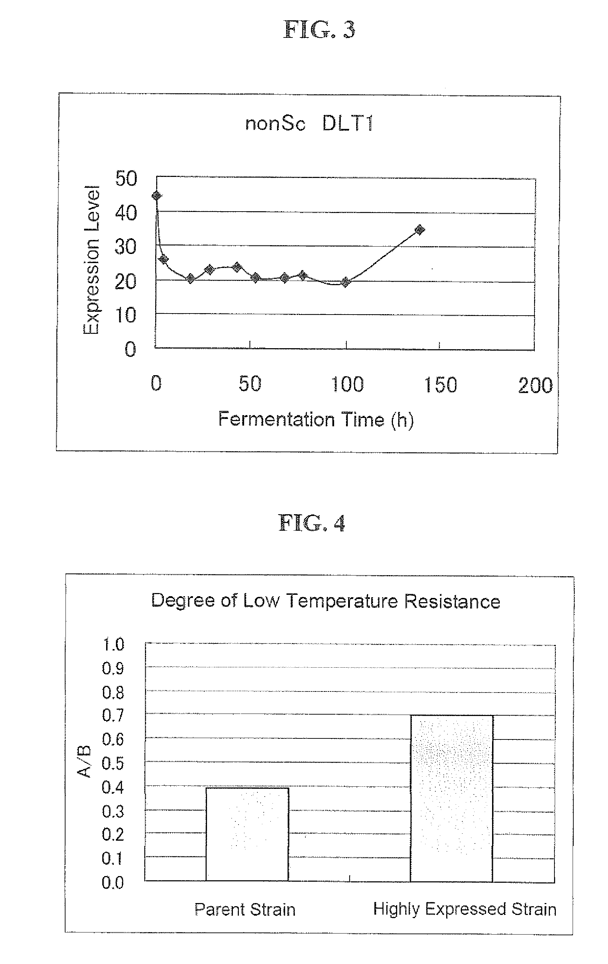

Sampling of fermentation liquor was performed with time, and variation with time of yeast growth amount (Fig. 1) and apparent extract concentration (Fig. 2) was observed. Simultaneously, sampling of yeast cells was performed, and the prepared mRNA was subjected to be biotin-labeled and was hybridized to a beer yeast DNA microarray described in Japanese Patent Application Laid-Open No. 2004-283169. The signal was detected using GCOS; GeneChip Operating Software 1.0 (manufactured by Affymetrix Co.). Expression pattern of non-ScDLT1 gene is shown in Figure 3. As a result, it was confirmed that non-ScDLT1 gene was expressed in the general beer fermentation.

### Example 3: Construction of non-ScDLT1 Gene Highly Expressed Strain

The non-ScDLT1/pCR2.1-TOPO described in Example 1 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the non-ScDLT1 high expression vector non-ScDLT1/pYCGPYNot. pYCGPYNot is the YCp-type yeast expression vector. The inserted gene is highly expressed by the pyruvate kinase gene PYK1 promoter. The geneticin-resistant gene G418^{r} is included as the selection marker in the yeast, and the ampicillin-resistant gene Amp^{r} is included as the selection marker in *Escherichia coli*.

Using the high expression vector prepared by the above method, AJL4004 strain was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin.

### Example 4: Assessment of Low Temperature Fermentability in Beer Fermentation

A fermentation test is carried out under the following conditions using the parent strain and the non-ScDLT1-highly expressed strain obtained in Example 3.

| | |
|---|---|
| Wort extract concentration | 12 % |
| Wort content | 1 L |
| Wort dissolved oxygen concentration | approx. 8 ppm |
| Fermentation temperature | 12°C (fixed) |
| Yeast pitching rate | 5 g wet yeast cells/L of wort |

The fermentation broth is sampled over time, and variation with time of the yeast growth rate (OD660), the amount of extract consumption and free amino nitrogen (FAN) is determined. The low temperature fermentability is assessed by measuring the ethanol production amount and the rate of ethanol production at 10 to 15°C.

### Example 5: Assessment of Low Temperature Resistance

The low temperature resistance of the parent strain and the highly expressed strain was assessed using the following method. The content of the medium used in the following example is as follows:
*YPD medium: Medium consisting of water containing 1% yeast extract, 2% Bacto peptone and 2% glucose
*YNB medium: Medium consisting of water containing 0.67% yeast nitrogen base, 0.4% casamino acid, 20ppm of uracil and 40ppm of adenine

Yeast cells (by a platinum loop) were seeded in 10mL of YPD medium containing 300mg/L of geneticin and were subjected to shaking culture at 30°C overnight. The turbidity of yeast cells (OD660) was measured, yeast cells corresponding to OD660=2 were collected, and thereafter the collected yeast cells were suspended in 1mL of YPD medium containing 4% glucose, 5% ethanol and 300mg/L of geneticin. Using the yeast suspension described above, a yeast suspension for freezing and a yeast suspension for non-freezirtg were prepared with respect to each of the parent strain and the highly expressed strain. To the yeast suspension for non-freezing, 1mL of YNB medium was added immediately, and was subjected to shaking culture at 30°C for 2 hours. The culture supernatant was collected by centrifugation, and the glucose concentration was measured using a biosensor BF-5 (manufactured by Oji Scientific Instruments). The yeast suspension for freezing was left to stand in an ice bath for 30 minutes for precooling and was frozen in a freezer at -20°C for 2 hours, and thereafter it was immersed in a water bath for 8 minutes to be thawed. 1mL of YNB medium was added thereto and the mixture was subjected to shaking culture at 30°C for 2 hours. After that, the glucose concentration was measured as in the case of the yeast suspension for non-freezing. The decrease amount of glucose in the yeast after freezing is designated as A, and the decrease amount of glucose in the non-frozen yeast is designated as B. The value of A/B is designated as "the degree of low temperature resistance". Thus, assessment of low temperature resistance was carried out.

As shown in Figure 4, the low temperature resistance of the parent strain was 0.4, while that of the highly expressed strain was 0.7. It became clear that the low temperature resistance is improved by high expression of non-SeDLT1.

### INDUSTRIAL APPLICABILITY

According to the method for producing alcoholic beverages of the present invention, low temperature performance of yeasts is improved. Therefore, it is possible to produce alcoholic beverages at a low temperature over a short period of time.

## Claims

1. A polynucleotide selected from the group consisting of:
(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one or more amino acids thereof being deleted, substituted, inserted and/or added, and having an activity to improve low temperature performance;
(d) a polynucleotide comprising a polynucleotide encoding a protein having an amino acid sequence having 60% or higher identity with the amino acid sequence of SEQ ID NO:2, and having an activity to improve low temperature performance;
(e) a polynucleotide comprising a polynucleotide which hybridizes to a polynucleotide consisting of a nucleotide sequence complementary to the nucleotide sequence of SEQ ID N0:1 under stringent conditions, and which encodes a protein having an activity to improve low temperature performance; and
(f) a polynucleotide comprising a polynucleotide which hybridizes to a polynucleotide consisting of a nucleotide sequence complementary to the nucleotide sequence of the polynucleotide encoding the protein of the amino acid sequence of SEQ ID NO:2 under stringent conditions, and which encodes a protein having an activity to improve low temperature performance.

2. The polynucleotide of Claim 1 selected from the group consisting of:
(g) a polynucleotide comprising a polynucleotide encoding a protein consisting of the amino acid sequence of SEQ ID NO: 2, or an amino acid sequence of SEQ ID NO: 2 wherein 1 to 10 amino acids thereof is deleted, substituted, inserted, and/or added, and wherein said protein has an activity to improve low temperature performance;
(h) a polynucleotide comprising a polynucleotide encoding a protein having an amino acid sequence which has 90% or higher identity with the amino acid sequence of SEQ ID NO: 2, and having an activity to improve low temperature performance; and
(i) a polynucleotide comprising a polynucleotide consisting of the nucleotide sequence of SEQ ID NO: 1 or a polynucleotide which hybridizes to a polynucleotide consisting of a nucleotide sequence complementary to the nucleotide sequence of SEQ ID NO: 1 under high stringent conditions, and which encodes a protein having an activity to improve low temperature performance.

3. The polynucleotide of Claim 1 comprising a polynucleotide consisting of SEQ ID NO: 1.

4. The polynucleotide of Claim 1 comprising a polynucleotide encoding a protein consisting of SEQ ID NO: 2.

5. The polynucleotide of any one of Claims 1 to 4, wherein the polynucleotide is DNA.

6. A protein encoded by the polynucleotide of any one of Claims 1 to 5.

7. A vector comprising the polynucleotide of any one of Claims 1 to 5.

8. A yeast, wherein the vector of Claim 7 is introduced.

9. The yeast of Claim 8, whose low temperature performance is improved by introducing the vector of Claim 7.

10. The yeast of Claim 9, whose low temperature performance is improved by increasing the expression level of the protein of Claim 6.

11. A method for producing an alcoholic beverage comprising culturing the yeast of any one of Claims 8 to 10.

12. The method for producing an alcoholic beverage of Claim 11, wherein the brewed alcoholic beverage is a malt beverage.

13. The method for producing an alcoholic beverage of Claim 11, wherein the brewed alcoholic beverage is wine.

14. An alcoholic beverage produced by the method of any one of Claims 11 to 13.

15. A method for assessing a test yeast for its low temperature performance, comprising using a primer or a probe designed based on a nucleotide sequence of a gene having the nucleotide sequence of SEQ ID NO: 1 and capable of improving low temperature performance.

16. A method for assessing a test yeast for its low temperature performance, comprising: culturing a test yeast; and measuring an expression level of a gene having the nucleotide sequence of SEQ ID NO: 1 and capable of improving low temperature performance.

17. A method for selecting a yeast, comprising: culturing test yeasts; quantifying the protein according to Claim 6 or measuring an expression level of a gene having the nucleotide sequence of SEQ ID NO: 1 and capable of improving low temperature performance; and selecting a test yeast having said protein amount or said gene expression level according to a target low temperature fermentability.

18. The method for selecting a yeast according to Claim 17, comprising: culturing a reference yeast and test yeasts; measuring an expression level of a gene having the nucleotide sequence of SEQ ID NO: 1 and capable of improving low temperature performance in each yeast; and selecting a test yeast having the gene expressed higher than that in the reference yeast.

19. The method for selecting a yeast according to Claim 17, comprising: culturing a reference yeast and test yeasts; quantifying the protein according to Claim 6 in each yeast; and selecting a test yeast having said protein in a larger amount than that in the reference yeast.

20. A method for producing an alcoholic beverage comprising: conducting fermentation for producing an alcoholic beverage using the yeast according to any one of Claims 8 to 10 or a yeast selected by the method according to any one of Claims 17 to 19; and improving low temperature performance.


SEQUENCE LISTING

<110>  Suntory Limited

<120>  Gene capable of improving low temperature fermentability and/or low
temperature resistance, and use thereof

<130>  G07-0100

<140>  PCT/JP2006/317699
<141>  2006-08-31

<150>  JP2005-253250
<151>  2005-09-01

<160>  4

<170>  PatentIn version 3.1

<210>  1
<211>  1029
<212>  DNA
<213>  yeast

<400>  1
atgtctgggc tagcgaaact gaagtcgtgg ctgtataagg gttccctctt tatatcattg      60
atacttttga ttgggttttc agtagtattg cctatagatt ccattgcaca ggcttctaaa     120
tcagagaaca atgctttcaa cacatttatt gttgttggtg ccttagttgt ttttggtgtt     180
gtttgtatcg ttattattat tggaagagtg ctatttcaca aaagctgtct aaaggatatt     240
ccaagaagat atattccaat cacaccagct gatcttccgc atcttgcgag tcgagaagca     300
gtattgaaaa acatggaaag gtctaaggaa ttaactattc tgctaaaaaa accaaaggat     360
cctgttatcc atgatggact ggaacctcca aagcgatgtg attttccatc aaatgaaaaa     420
ttatttccag aatacctaaa ttatgctgat tgtataaaaa gtctaacgga tagattgaaa     480
taccatgggt tgtttctgaa caacctagat gttaggatga aactggagga cacttttgct     540
gatgtggtga attctcagtt tgttaaccgc aatactaaca aggttcaatt gcaaaaggct     600
aaagatttta ttgacttgta tgaaacgata agattttcag gcaaagatgt cacaagagac     660

caatttatac tgtttgttga gctgtgtctt tattttgggg aggtgtcact aacaagggat 720
acgtcatttg caaatttcca gaattttaaa tataatgcca gttcaaataa tggcggaaca 780
aatgaatcga aatattcaat aaaccggttc gatgaaaacg aatacgctca ggaagatatg 840
cattactttc ctgaaccgcc tacccatttg gttagagaga gtagcagaag cacagtggca 900
cggcatattt catctggtgg agatttacct aattctgaag agcatccttt agaagacgat 960
tctgattgta atgggcttaa tgacaaactt gcagaagttc acagttatag aagcgtaatt 1020
cgtcattaa
1029

<210> 2
<211> 342
<212> PRT
<213> yeast

<400> 2

Met Ser Gly Leu Ala Lys Leu Lys Ser Trp Leu Tyr Lys Gly Ser Leu
1               5                   10                  15

Phe Ile Ser Leu Ile Leu Leu Ile Gly Phe Ser Val Val Leu Pro Ile
            20                  25                  30

Asp Ser Ile Ala Gln Ala Ser Lys Ser Glu Asn Asn Ala Phe Asn Thr
        35                  40                  45

Phe Ile Val Val Gly Ala Leu Val Val Phe Gly Val Val Cys Ile Val
    50                  55                  60

Ile Ile Ile Gly Arg Val Leu Phe His Lys Ser Cys Leu Lys Asp Ile
65                  70                  75                  80

Pro Arg Arg Tyr Ile Pro Ile Thr Pro Ala Asp Leu Pro His Leu Ala
                85                  90                  95

Ser Arg Glu Ala Val Leu Lys Asn Met Glu Arg Ser Lys Glu Leu Thr
            100                 105                 110

Ile Leu Leu Lys Lys Pro Lys Asp Pro Val Ile His Asp Gly Leu Glu
        115                 120                 125

Pro Pro Lys Arg Cys Asp Phe Pro Ser Asn Glu Lys Leu Phe Pro Glu
    130                 135                 140

Tyr Leu Asn Tyr Ala Asp Cys Ile Lys Ser Leu Thr Asp Arg Leu Lys
145                 150                 155                 160

Tyr His Gly Leu Phe Leu Asn Asn Leu Asp Val Arg Met Lys Leu Glu
                165                 170                 175

Asp Thr Phe Ala Asp Val Val Asn Ser Gln Phe Val Asn Arg Asn Thr
            180                 185                 190

Asn Lys Val Gln Leu Gln Lys Ala Lys Asp Phe Ile Asp Leu Tyr Glu
        195                 200                 205

Thr Ile Arg Phe Ser Gly Lys Asp Val Thr Arg Asp Gln Phe Ile Leu
    210                 215                 220

Phe Val Glu Leu Cys Leu Tyr Phe Gly Glu Val Ser Leu Thr Arg Asp
225                 230                 235                 240

Thr Ser Phe Ala Asn Phe Gln Asn Phe Lys Tyr Asn Ala Ser Ser Asn
                245                 250                 255

Asn Gly Gly Thr Asn Glu Ser Lys Tyr Ser Ile Asn Arg Phe Asp Glu
            260                 265                 270

Asn Glu Tyr Ala Gln Glu Asp Met His Tyr Phe Pro Glu Pro Pro Thr
        275                 280                 285

His Leu Val Arg Glu Ser Ser Arg Ser Thr Val Ala Arg His Ile Ser
    290                 295                 300

Ser Gly Gly Asp Leu Pro Asn Ser Glu Glu His Pro Leu Glu Asp Asp
305                 310                 315                 320

Ser Asp Cys Asn Gly Leu Asn Asp Lys Leu Ala Glu Val His Ser Tyr
                325                 330                 335

Arg Ser Val Ile Arg His
            340

<210>  3
<211>  40
<212>  DNA
<213>  primer

<400>  3
gagctcatag cggccatgtc tgggctagcg aaactgaagt                              40

<210>  4
<211>  42
<212>  DNA
<213>  primer

<400>  4
ggatcctatg cggccgctat ttttaggatt atgattattc aa                           42